# EUROPÄISCHE PATENTSCHRIFT

(11) **EP 1 658 016 B1**
(45) Veröffentlichungstag und Bekanntmachung des Hinweises auf die Patenterteilung: **22.01.2014**
(21) Anmeldenummer: 04762993.6
(22) Anmeldetag: 09.06.2004
(51) Int. Cl.: A61B 19/00

(54) **CHIRURGISCHE HALTEVORRICHTUNG**
SURGICAL SUPPORT
DISPOSITIF SUPPORT CHIRURGICAL

(30) Priorität: 26.08.2003 DE 10340151
(43) Veröffentlichungstag der Anmeldung: 24.05.2006
(73) Patentinhaber: Aesculap AG, 78532 Tuttlingen (DE); RWTH Aachen, 52062 Aachen (DE)
(72) Erfinder: FISCHER, Manfred, 73479 Ellwangen (DE); LUTZE, Theodor, 78582 Balgheim (DE); RADERMACHER, Klaus, 52222 Stolberg (DE); HAHNDORF, Markus, 52064 Aachen (DE); LAUER, Wolfgang, 52066 Aachen (DE)
(74) Vertreter: Hoeger, Stellrecht & Partner Patentanwälte
(86) Internationale Anmeldenummer: PCT/EP2004/006209
(87) Internationale Veröffentlichungsnummer: WO 2005/030074

(56) Entgegenhaltungen:
- WO-A-03/009768
- US-A- 5 697 939
- US-B1- 6 179 262
- US-B1- 6 368 332

## Beschreibung

Die Erfindung betrifft eine chirurgische Haltevorrichtung zum Halten eines chirurgischen Instruments, mit einem Rahmen, mit einer mindestens zwei Lenker umfassenden ersten Lenkeranordnung, welchen den Rahmen mit einem dem Instrument zugeordneten ersten Gelenkpunkt gelenkig verbindet und mit einer mindestens zwei Lenker umfassenden zweiten Lenkeranordnung, welche den Rahmen mit einem dem Instrument zugeordneten zweiten Gelenkpunkt gelenkig verbindet, wobei die Lenker der ersten Lenkeranordnung relativ zueinander und relativ zum Rahmen um Schwenkachsen schwenkbar gelagert sind und die Schwenkachsen parallel zueinander verlaufen und wobei die beiden Lenkeranordnungen so ausgebildet sind, daß der erste Gelenkpunkt in einer ersten Bewegungsebene und der zweite Gelenkpunkt in einer zweiten Bewegungsebene bewegbar ist.

Eine chirurgische Haltevorrichtung zum Halten eines chirurgischen Instruments, mit einem Rahmen, mit einer mindestens zwei Lenker umfassenden ersten Lenkeranordnung, welchen den Rahmen mit einem dem Instrument zugeordneten ersten Gelenkpunkt gelenkig verbindet und mit einer mindestens zwei Lenker umfassenden zweiten Lenkeranordnung, welche den Rahmen mit einem dem Instrument zugeordneten zweiten Gelenkpunkt gelenkig verbindet ist beispielsweise aus der US 2002/0038118 A1 bekannt, die zwei starr mit einer Basis verbundene Lenker und zwei relativ zu den an der Basis gehaltenen Lenkern bewegbare Lenker umfaßt. Die an der Basis gehaltenen Lenker umfassen einen Translationsantrieb. Translationsantriebe haben den Nachteil, daß sie relativ groß und schwer sind, so daß der feststehende Lenker besonders stabil ausgebildet werden muß. Mit der bekannten Vorrichtung lassen sich ferner die beiden Gelenkpunkte nur in einem sehr eng begrenzten Bereich positionieren, das heißt ein Aktionsradius des chirurgischen Instruments ist eng begrenzt.

Weitere chirurgische Haltevorrichtungen sind aus der US 5,697,939, aus der US 6,179,262 B1, aus der US 6,368,332 B1 sowie aus der WO 03/009768 A1 bekannt.

Daher ist es Aufgabe der vorliegenden Erfindung, eine chirurgische Haltevorrichtung der eingangs beschriebenen Art so zu verbessern, daß möglichst einfach ein großer Aktionsbereich des mit der Haltevorrichtung gehaltenen chirurgischen Instruments einstellbar ist.

Diese Aufgabe wird bei einer chirurgischen Haltevorrichtung der eingangs beschriebenen Art erfindungsgemäß dadurch gelöst, daß die erste Bewegungsebene relativ zur zweiten Bewegungsebene bewegbar ist.

Gemäß der vorgeschlagenen Anordnung sind alle Lenker in ihrer Position relativ zum Rahmen verstellbar, das heißt, daß auch ein am Rahmen gehaltener Lenker relativ zu diesem schwenkbar gelagert ist. Auf diese Weise läßt sich der erste Gelenkpunkt in einem Aktionsbereich bewegen, welcher eine maximale Fläche mit einer maximalen Seitenlänge aufweist, welche einer Gesamtlänge der hintereinander angeordneten Lenker entspricht. Die parallele Anordnung der Schwenkachsen ermöglicht auch eine Bewegung des ersten Gelenkpunkts in einer Ebene. Vorteilhaft ist es, dass die beiden Lenkeranordnungen so ausgebildet sind, daß der erste Gelenkpunkt in einer ersten Bewegungsebene und der zweite Gelenkpunkt in einer zweiten Bewegungsebene bewegbar ist. Unabhängig von einer Ausgestaltung der beiden Gelenkpunkte lassen sich so Bewegungen der beiden Gelenkpunkte auf besonders einfache Weise ausführen und auch überwachen, beispielsweise indem die Haltevorrichtung in Verbindung mit einem Navigationssystem eingesetzt wird. Denkbar wäre es, daß die erste Bewegungsebene relativ zur zweiten Bewegungsebene unveränderlich ist. Vorteilhaft ist es jedoch, dass die erste Bewegungsebene relativ zur zweiten Bewegungsebene bewegbar ist. Dies ermöglicht es, den Abstand zwischen den beiden Gelenkpunkten konstant zu halten und eine sonst bei einer Veränderung von Stellungen der Lenkeranordnungen erforderliche Veränderung des Abstands zwischen beiden Gelenkpunkten anzupassen.

Günstig ist es, wenn die Lenker der zweiten Lenkeranordnung relativ zueinander und relativ zum Rahmen um Schwenkachsen schwenkbar gelagert sind und wenn die Schwenkachsen parallel zueinander verlaufen. Damit läßt sich auf einfache Weise der zweite Gelenkpunkt in einer Ebene senkrecht zu den Schwenkachsen bewegen. Der Aktionsbereich des zweiten Gelenkpunkts entspricht im wesentlichen einer Fläche mit einer Seitenlänge, welche einer Länge der hintereinander angeordneten Lenker der zweiten Lenkeranordnung entspricht.

Besonders einfach wird der Aufbau der Vorrichtung, wenn die erste Bewegungsebene relativ zur zweiten Bewegungsebene verschwenkbar ist.

Vorteilhaft ist es ferner, wenn ein Abstand zwischen der ersten Bewegungsebene und der zweiten Bewegungsebene veränderbar ist. Auf diese Weise läßt sich ein Neigungswinkelbereich einer Längsachse des Instruments relativ zu einer der Schwenkachsen zusätzlich vergrößern.

Vorteilhaft ist es, wenn der Rahmen ein erstes und mindestens ein zweites Rahmenelement umfaßt und wenn die erste Lenkeranordnung am ersten Rahmenelement und die zweite Lenkeranordnung am zweiten Rahmenelement angeordnet ist. Damit könnte man beispielsweise die Haltevorrichtung an zwei verschiedenen Stativelementen befestigen, was insbesondere bei manchen chirurgischen Eingriffen räumliche Vorteile bietet.

Für eine möglichst optimale Verstellbarkeit der Anordnung kann das erste Rahmenelement relativ zum zweiten Rahmenelement bewegbar gelagert sein.

Die Rahmenelemente können direkt aneinander gelagert sein oder aber auch an voneinander getrennten Orten angeordnet sein.

Ein besonders einfacher Aufbau der Vorrichtung ergibt sich, wenn das erste Rahmenelement relativ zum zweiten Rahmenelement um eine Drehachse verschwenkbar ist. Eine derartige Schwenklagerung läßt sich auf einfache Weise realisieren.

Auch wenn es denkbar wäre, die Drehachse rechtwinklig oder schräg zu den beiden Bewegungsebenen vorzusehen, ist es besonders vorteilhaft, wenn die Drehachse parallel zu den beiden Bewegungsebenen verläuft. Damit läßt sich auf einfache Weise eine Schwenkbewegung der beiden Bewegungsebenen relativ zueinander realisieren.

Günstig ist es, wenn das erste Rahmenelement relativ zum zweiten Rahmenelement in einer Richtung quer zu der ersten Bewegungsebene verschiebbar ist. Damit läßt sich ein Abstand zwischen den beiden Rahmenelementen verändern, wodurch auch ein Abstand zwischen den beiden Bewegungsebenen veränderbar ist. Dadurch läßt sich wiederum eine erforderliche Abstandsänderung zwischen den Gelenkpunkten ausgleichen, was erforderlich ist, wenn die beiden Gelenkpunkte in einem festen Abstand voneinander angeordnet sein sollen.

Gemäß einer bevorzugten Ausführungsform der Erfindung kann vorgesehen sein, daß die erste Lenkeranordnung fünf relativ zueinander in der ersten Bewegungsebene verschwenkbare Lenker umfaßt. Mit fünf derartig gelagerten Lenkern läßt sich der erste Gelenkpunkt in der ersten Bewegungsebene wunschgemäß positionieren.

Günstig ist es, wenn die fünf Lenker der ersten Lenkeranordnung so miteinander verbunden sind, daß sie eine geschlossene erste Ringstruktur bilden. Diese kann beispielsweise dadurch gebildet werden, daß jeder Lenker ein erstes und ein zweites Ende aufweist und daß ein erstes Ende eines Lenkers mit einem zweiten Ende eines anderen Lenkers verbunden ist.

Um die Vorrichtung besonders kompakt auszubilden, ist es vorteilhaft, wenn das erste Rahmenelement einen der ersten fünf Lenker der ersten Lenkeranordnung umfaßt. Dies bedeutet, daß zwei Lenker am ersten Rahmenelement schwenkbar gelagert sind.

Vorteilhaft ist es, wenn der erste Gelenkpunkt mindestens einem der Lenker der ersten Lenkeranordnung zugeordnet ist. Er kann beispielsweise direkt am Lenker angeordnet sein oder mittels eines weiteren Elements von diesem beabstandet sein. In jedem Fall ist es günstig, wenn eine Relativposition des ersten Gelenkpunkts relativ zu dem mindestens einen Lenker der ersten Lenkeranordnung konstant bleibt.

Um eine optimale Positionierung des zweiten Gelenkpunkts in der zweiten Bewegungsebene zu erreichen, ist es günstig, wenn die zweite Lenkeranordnung fünf relativ zueinander in der zweiten Bewegungsebene verschwenkbare Lenker umfaßt. Damit lassen sich ferner besonders kompakte und langgestreckte Lenkeranordnungen aufbauen. Eine Länge der Lenker kann je nach Bedarf variieren.

Um eine besonders gute Führung für den zweiten Gelenkpunkt zu erhalten, ist es günstig, wenn die fünf Lenker der zweiten Lenkeranordnung derart miteinander verbunden sind, daß sie eine geschlossene Ringstruktur bilden. Wird beispielsweise der zweite Gelenkpunkt durch eine Verbindung zwischen zwei Lenkern definiert, so wird der zweite Gelenkpunkt beidseitig gehalten und geführt.

Besonders kompakt kann die Vorrichtung aufgebaut werden, wenn das zweite Rahmenelement einen der fünf Lenker der zweiten Lenkeranordnung umfaßt. Eine derartige Anordnung könnte beispielsweise so gestaltet sein, daß zwei der fünf Lenker am zweiten Rahmenelement schwenkbar gelagert sind.

Eine definierte Positionierung des zweiten Gelenkpunkts ist möglich, wenn der zweite Gelenkpunkt mindestens einem der Lenker der zweiten Gelenkanordnung zugeordnet ist. Dies könnte eine starre oder auch eine in einem Abstand veränderliche Zuordnung sein.

Ohne zusätzliche Gelenke kommt die Vorrichtung aus, wenn die Schwenkachsen der Lenker der ersten Lenkeranordnung rechtwinklig zur ersten Bewegungsebene verlaufen.

Vorteilhaft ist es, wenn die Schwenkachsen der Lenker der zweiten Lenkeranordnung rechtwinklig zur zweiten Bewegungsebene verlaufen. Auf diese Weise läßt sich eine Bewegung des zweiten Gelenkpunkts in der zweiten Bewegungsebene führen und dessen Position halten.

Grundsätzlich wäre es denkbar, die Lenker der beiden Lenkeranordnungen von Hand in eine gewünschte Position zu bringen. Es ist jedoch vorteilhaft, wenn mindestens ein Lenkerantrieb zum Bewegen mindestens eines am Rahmen gelagerten Lenkers einer der beiden Lenkeranordnungen vorgesehen ist. Dies ermöglicht eine Positionierung des ersten Gelenkpunkts unabhängig von einem manuellen Eingriff einer Bedienperson. Beispielsweise kann auf diese Weise eine Position eines chirurgischen Instruments in einem sterilen Bereich verändert werden, ohne daß eine Bedienperson in den sterilen Bereich eingreifen muß.

Um eine vollständig automatische Einstellung von Positionen der beiden Gelenkpunkte im Raum zu ermöglichen, ist es günstig, wenn jeder der beiden Lenkeranordnungen zwei Lenkerantriebe zugeordnet sind. Sind alle Lenker der Lenkeranordnung miteinander verbunden, so ergibt sich beispielsweise bei fünf Lenkern eine Zwangsführung zweier nicht angetriebener Lenker, wenn die angetriebenen Lenker an einem feststehenden Lenker angeordnet sind.

Denkbar wäre es, Linearantriebe als Lenkerantriebe einzusetzen. Vorteilhaft ist es jedoch, wenn der mindestens eine Lenkerantrieb ein Rotationsantrieb zum Schwenken des mindestens einen am Rahmen gelagerten Lenkers relativ zum Rahmen ist. Damit läßt sich eine besonders kompakte Bauform der Vorrichtung erreichen, denn der Antrieb muß nicht im Lenker integriert werden. Dadurch können die Lenker besonders schlank und leicht ausgebildet werden. Außerdem sind drehgelenkige Rotationsantriebe weniger spielbehaftet als Linearantriebe.

Denkbar wäre es, den Lenkerantrieb an einem Lenker anzuordnen. Vorzugsweise ist der mindestens eine Lenkerantrieb jedoch am Rahmen angeordnet. Dadurch ist es möglich, den Lenkerantrieb außerhalb eines Operationsbereichs anzuordnen, was einen Zugang zum Operationsbereich deutlich erleichtert.

Um eine roboterähnliche Haltevorrichtung zu realisieren, ist es vorteilhaft, wenn die Lenkerantriebe unabhängig voneinander ansteuerbar sind. Auf diese Weise können die beiden Gelenkpunkte unabhängig voneinander in jede gewünschte Position gebracht werden, die mit der Vorrichtung einstellbar ist.

Denkbar wäre es, die beiden Gelenkpunkte direkt an den beiden Lenkeranordnungen vorzusehen, beispielsweise jeweils an einem Lenker oder an einem zwei Lenker verbindenden Gelenkpunkt. Vorteilhaft ist es jedoch, wenn jede der beiden Lenkeranordnungen ein Halteelement trägt und wenn jedem Halte-element einer der beiden Gelenkpunkte zugeordnet ist. Beispielsweise kann so auf einfache Weise eine Trennung zwischen einem sterilen und einem nicht sterilen Bereich erfolgen, der das Halteelement in zwei Halteelementabschnitte trennt, nämlich einen sterilen und einen nicht sterilen. Außerdem lassen sich mit dem Halteelement große Distanzen zwischen der Lenkeranordnung und dem Instrument vorgeben.

Um einen Abstand zwischen dem Instrument und der Lenkeranordnung zu variieren, kann das mindestens eine Halteelement lösbar mit einem der Lenker verbindbar sein.

Um die Vorrichtung beispielsweise teilweise in einem nicht sterilen Bereich anzuordnen, das Instrument jedoch in einem sterilen Bereich zu halten, kann jedes Halteelement eine Sterilschnittstelle umfassen. Dies ermöglicht es, das Halteelement teilweise in dem sterilen Bereich anzuordnen, wo es das Instrument hält, teilweise im nicht sterilen Bereich, in dem die Lenkeranordnung und auch etwaige Lenkerantriebe angeordnet sein können.

Besonders kompakt wird die Lenkeranordnung, wenn mindestens eine der beiden Lenkeranordnungen zwei sich überkreuzende Lenker umfaßt.

Besonders einfach wird der Aufbau der Vorrichtung dann, wenn die Vorrichtung nur Drehgelenke zum Bewegen der Lenker und der Gelenkpunkte relativ zueinander umfaßt. Drehgelenke lassen sich besonders einfach ausbilden, sind nahezu spielfrei und ermöglichen beispielsweise in Kombination auch eine Bewegung um mehr als nur einen Freiheitsgrad.

Um eine nahezu beliebige Einstellung einer Position des Instruments zu gestatten, ist es günstig, wenn der erste und/oder der zweite Gelenkpunkt ein Mehrfreiheitsgradgelenk umfaßt.

Vorteilhaft ist es, wenn das Mehrfreiheitsgradgelenk ein Kugelgelenk ist. Eine Kugelgelenklagerung des Instruments an der Haltevorrichtung ermöglicht eine nahezu beliebige Orientierung einer Längsachse des Instruments im Raum. Außerdem läßt sich das Gelenk auf diese Weise besonders kompakt ausbilden.

Günstig ist es, wenn das Mehrfreiheitsgradgelenk in Form eines Kreuzgelenks ausgebildet ist. Ein solches Gelenk kann allein durch Drehgelenke gebildet werden.

Falls die beiden Bewegungsebenen relativ zueinander nicht verschiebbar sind, ist es günstig, wenn das Instrument an mindestens einem der beiden Gelenkpunkte verschiebbar gehalten ist. Damit lassen sich Abstände zwischen den beiden Gelenkpunkten beliebig einstellen.

Grundsätzlich könnte das Instrument direkt an der Haltevorrichtung gehalten sein. Vorteilhaft ist es jedoch, wenn eine Führung für das Instrument vorgesehen ist und wenn die beiden Gelenkpunkte an der Führung angeordnet sind. Auf diese Weise kann die Führung im Raum positioniert und auf einfache Weise das Instrument von dieser aufgenommen werden. Ferner lassen sich Instrumente besonders einfach austauschen, nämlich indem sie aus der Führung entnommen und durch andere Instrumente ersetzt werden.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Ansicht einer erfindungsgemäßen Haltevorrichtung von vorne oben;
- Figur 2:: eine perspektivische Ansicht der Haltevorrichtung von hinten;
- Figur 3:: eine Ansicht der Haltevorrichtung von oben;
- Figur 4:: eine Seitenansicht der Haltevorrichtung aus Figur 3;
- Figur 5:: eine Draufsicht auf eine Haltevorrichtung mit zueinander veränderten Lenkerpositionen;
- Figur 6:: eine Seitenansicht der Haltevorrichtung aus Figur 5; und
- Figur 7:: ein zweites Ausführungsbeispiel einer erfindungsgemäßen Haltevorrichtung.

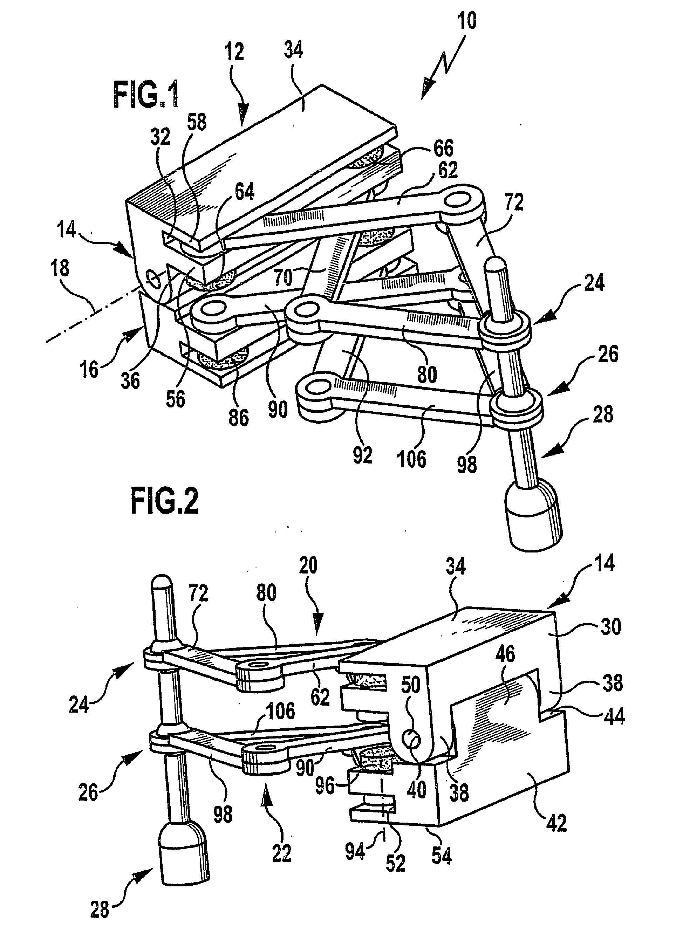

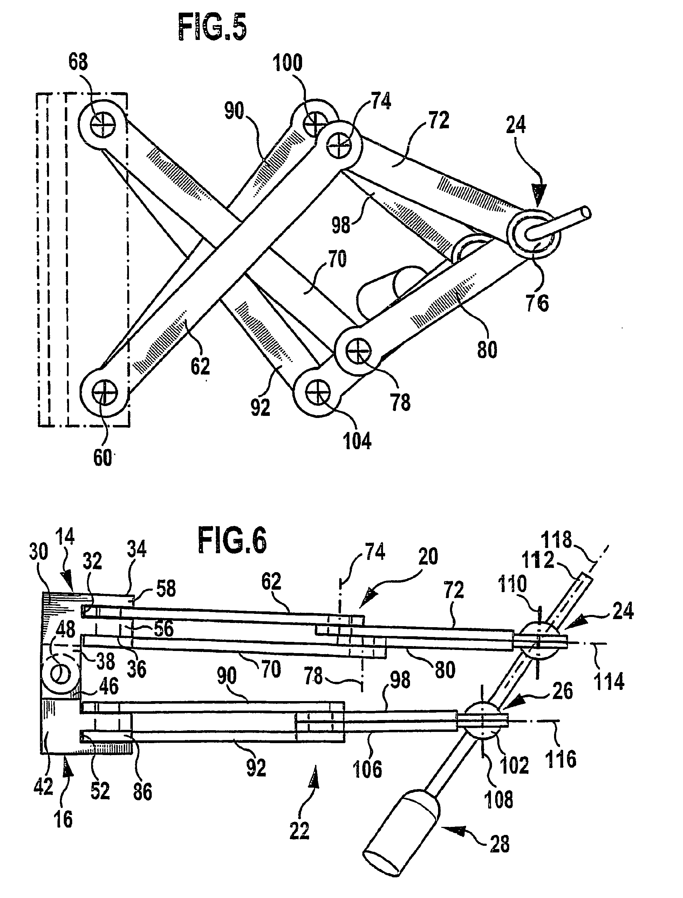

In den Figuren 1 bis 6 ist eine insgesamt mit dem Bezugszeichen 10 versehene, modular an verschiedene chirurgische Haltesysteme ankoppelbare, motorisch angetriebene Geräteträgerplattform dargestellt. Sie umfaßt einen zweiteiligen Rahmen 12 mit einer oberen Rahmenhälfte 14 und eine um eine Rahmenschwenkachse 18 relativ zur oberen Rahmenhälfte 14 schwenkbar gelagerte untere Rahmenhälfte 16. Jeder der beiden Rahmenhälften 14 und 16 ist ein fünfgliedriges Kurbelgetriebe 20 beziehungsweise 22 zugeordnet, welche beim vorliegenden Ausführungsbeispiel identisch aufgebaut sind. Ferner trägt jedes der beiden Kurbelgetriebe 20 und 22 einen Anlenkpunkt 24 beziehungsweise 26, an welchen ein chirurgisches Instrument 28, beispielsweise in Form eines Fräswerkzeugs oder einer Endoskopoptik, gehalten ist.

Die obere Rahmenhälfte 14 umfaßt einen quaderförmigen Grundkörper 30, der auf einer seiner schmalen langen Seiten mit einer Längsnut 32 versehen ist, die parallel zu einer Oberseite 34 des Grundkörpers 30 angeordnet ist. Von einer Unterseite 36 des Grundkörpers 30 stehen rechtwinklig zwei Lagerbolzen 38 ab, die jeweils mit einer Bohrung 40 versehen sind.

Die untere Rahmenhälfte 16 weist einen ebenfalls quaderförmigen Grundkörper 42 auf, von dessen Oberseite 44 ein Lagerbolzen 46 rechtwinklig absteht, der ebenfalls mit einer Bohrung 48 versehen ist. Der Lagerbolzen 46 ist zwischen die beiden Lagerbolzen 38 eingefügt und wird zwischen diesen beiden in Richtung der Rahmenschwenkachse 18 unverschieblich gehalten. Zu diesem Zweck ist eine zylindrische Lagerwelle 50 in die Bohrungen 40 und 48 eingeschoben und drehfest mit den Lagerbolzen 38 verbunden. Eine parallel zur Unterseite 54 des Grundkörpers 42 angeordnete Längsnut 52 ist in Richtung von einer schmalen langen Seitenfläche des Grundkörpers 30 weg offen.

In einer relativen Stellung der Rahmenhälfte 14 zur Rahmenhälfte 16, in welcher die Oberseite 34 parallel zur Unterseite 54 verläuft, sind beide Längsnuten 32 und 52 parallel zueinander und in die gleiche Richtung weisend ausgerichtet.

Das Kurbelgetriebe 20 umfaßt einen ersten Lenker, der durch eine Nutseitenwand 56 der Längsnut 32 gebildet wird. In der Längsnut 32 ist an einem Ende derselben zwischen der Nutseitenwand 56 und einer Nutseitenwand 58 ein flacher rechteckiger Lenker 62 schwenkbar um eine rechtwinklig zur Oberseite 34 verlaufende Drehachse 60 angeordnet. Er ist mit einem Rotationsantrieb 64 um die Drehachse 60 schwenkbar, wobei der Rotationsantrieb 64 auch rotationssymmetrisch zur Drehachse 60 auf der Unterseite 36 angeordnet ist.

In der Längsnut 32 ist an deren anderem Ende zwischen den Nutseitenwänden 56 und 58 ein zweiter Rotationsantrieb 66 angeordnet, der einen um eine zur Oberseite 34 rechtwinklig verlaufende Drehachse 68 schwenkbar gelagerten langgestreckten rechteckigen Lenker 70 antreibt. Ein freies Ende des Lenkers 62 ist gelenkig und um eine Drehachse 74 schwenkbar mit einem weiteren langgestreckten rechteckigen Lenker 72 verbunden, der etwas kürzer als der Lenker 62 ist. Ein freies Ende des Lenkers 72 umgreift eine Gelenkkugel 76. Ein freies Ende des Lenkers 70 ist gelenkig und um eine Drehachse 78 verschwenkbar mit einem fünften Lenker 80 verbunden, der ein die Gelenkkugel 76 umgreifendes Ende aufweist. Insgesamt umfaßt das fünfgliedrige Kurbelgetriebe 20 somit fünf Lenker, nämlich die Nutseitenwand 56, sowie die Lenker 62, 72, 80 und 70.

Das zweite fünfgliedrige Kurbelgetriebe 22 umfaßt als feststehenden Lenker eine erste, in Richtung auf die obere Rahmenhälfte 14 weisende Nutseitenwand 82 der Längsnut 52, die ferner eine untere Nutseitenwand 84 aufweist. An einem Ende der Längsnut 52 ist zwischen den Nutseitenwänden 82 und 84 ein Rotationsantrieb 86 symmetrisch zu einer Drehachse 88 angeordnet und dient zum Antreiben eines an der Nutseitenwand 82 um die Drehachse 88 schwenkbar gelagerten, langgestreckten quaderförmigen Lenkers 90. Zwischen den beiden Nutseitenwänden 82 und 84 ist an einem anderen Ende der Längsnut 52 ein weiterer langgestreckter quaderförmiger Lenker 92 um eine Drehachse 94 schwenkbar gelagert. Der Lenker 92 wird angetrieben durch einen insgesamt vierten Rotationsantrieb 96, der symmetrisch zur Drehachse 94 angeordnet ist. Ein freies Ende des Lenkers 90 ist an einem etwas kürzeren, langgestreckten quaderförmigen Lenker 98 um eine Drehachse 100 schwenkbar gelagert. Ein freies Ende des Lenkers 98 umgreift eine Gelenkkugel 102 des Anlenkpunkts 26. Ein freies Ende des Lenkers 92 ist um eine Drehachse 104 schwenkbar mit einem Lenker 106 verbunden, welcher ein freies, die Gelenkkugel 102 umgreifendes Ende aufweist. Im Bereich des Anlenkpunkts 26 definieren die beiden Lenker 90 und 106 somit eine weitere Drehachse 108, ebenso wie die beiden Lenker 72 und 78 im Bereich des Anlenkpunkts 24 eine gemeinsame Drehachse 110 definieren.

Wahlweise können die Lenker 62, 70, 90 und 92 auch jeweils direkt mittels einer nicht dargestellten Antriebsschnecke angetrieben werden.

Das Instrument 28 ist an den beiden Anlenkpunkten 24 und 26 an den Gelenkkugeln 76 und 102 drehfest gehalten. Eine Rotation eines Schafts 112 des Instruments 28 um eine Längsachse 118 ist möglich, denn die Gelenkkugeln 76 und 102 sind an den Lenkern 72 und 80 beziehungsweise 98 und 106 frei rotierbar gelagert.

Jedes Kurbelgetriebe 20 beziehungsweise 22 weist zwei sich kreuzende Lenker auf, nämlich die Lenker 62 und 70 beim Kurbelgetriebe 20 und die Lenker 90 und 92 beim Kurbelgetriebe 22.

Das Kurbelgetriebe 22 ist insgesamt fünfgliedrig ausgebildet und umfaßt die Nutseitenwand 82 sowie die Lenker 90, 98, 106 und 92.

Die Drehachsen 60, 74, 110, 78 und 68 des Kurbelgetriebes 20 sind alle parallel zueinander und senkrecht zur Oberseite 34 der oberen Rahmenhälfte 14 orientiert. Die Drehachsen 88, 100, 108, 104 und 94 sind ebenfalls parallel zueinander und rechtwinklig zur Unterseite 54 der unteren Rahmenhälfte 16 ausgerichtet. Dadurch kann der Anlenkpunkt 24 in einer ersten Ebene 114 und der zweite Anlenkpunkt 26 in einer zweiten Ebene 116 mittels der beiden Kurbelgetriebe 20 beziehungsweise 22 bewegt werden.

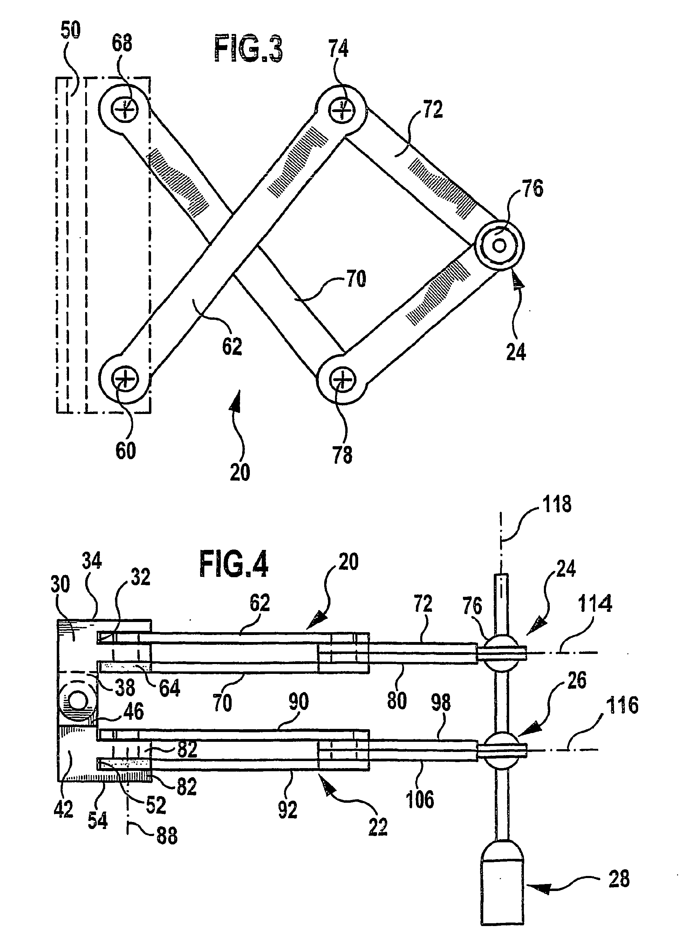

Der Lenker 62 ist mittels des Rotationsantriebs 64 frei verschwenkbar. Ebenso ist der Lenker 70 mittels des Rotationsantriebs 66 verschwenkbar. Da freie Enden der Lenker 72 und 80 miteinander um die Drehachse 110 schwenkbar verbunden sind, ergibt sich eine Zwangsführung für den Anlenkpunkt 24. Dieser entfernt sich um so weiter vom Rahmen 12, je paralleler die Lenker 62 und 70 zueinander ausgerichtet werden. Sind die beiden Kurbelgetriebe 20 und 22, wie in den Figuren 1 bis 6 dargestellt, in ihrem Aufbau identisch, und sind sie zunächst in ihren Relativpositionen identisch eingestellt, so verläuft die Längsachse 118 des Schafts 112 zunächst parallel zur Drehachse 110. Diese Stellung ist in den Figuren 3 und 4 dargestellt.

Bleibt das Kurbelgetriebe 22 unverändert, werden jedoch die Lenker 62 und 70 relativ zueinander verschwenkt, so daß die Drehachsen 74 und 78 enger zusammenrücken, so wird der Anlenkpunkt 24 vom Rahmen 12 weg bewegt. Dadurch neigt sich die Längsachse 118 des Schafts 112 relativ zur Drehachse 110. Da der Abstand zwischen den Gelenkkugeln 76 und 102 fest vorgegeben und daher konstant ist, da der Schaft 112 drehfest mit den Gelenkkugeln 76 und 102 verbunden ist, neigt sich die Rahmenhälfte 14 relativ zur Rahmenhälfte 16 durch Verschwenken um die Rahmenschwenkachse 18.

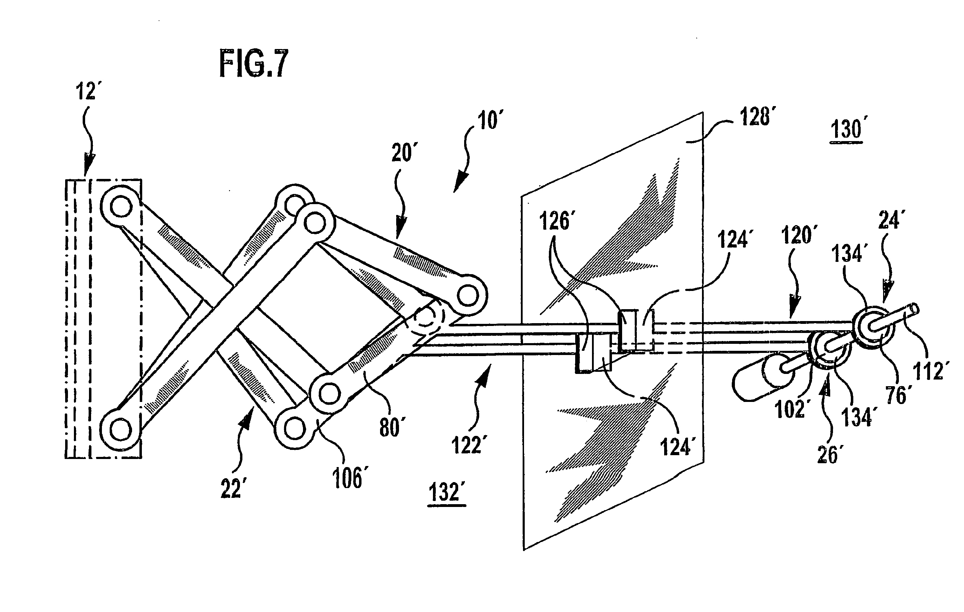

In Figur 7 ist eine insgesamt mit dem Bezugszeichen 10' leicht modifizierte Geräteträgerplattform dargestellt. Zwei Kurbelgetriebe 20' und 22' sind in oben beschriebener Weise an einem Rahmen 12' gehalten. Vom Lenker 80' steht schräg ein insgesamt mit dem Bezugszeichen 120' versehener zweiteiliger Abstandshalter ab, in ähnlicher Weise vom Lenker 106' ein Abstandshalter 122'. Die Abstandshalter 120' und 122' sind in Form langgestreckter Stäbe ausgebildet, die in etwa mittig über eine zweiteilige Steckverbindung umfassend einen Stecker 124' und eine Kupplung 126' miteinander verbunden sind. Zwischen die Stecker 124' und die Kupplung 126' ist eine Sterilfolie 128' einklemmbar, die einen Sterilbereich 130' von einem nicht sterilen Bereich 132' trennt. Freie Enden der Abstandshalter 120' und 122' tragen die Anlenkpunkte 24' und 26', welche durch an einem freien Ende der Abstandhalter 120' und 122' gehaltene Lagerringe 134' und von diesen umgebene Gelenkkugeln 76' und 102' definiert werden. Der Schaft 112' des Instruments 28' ist drehfest mit den Gelenkkugeln 76' und 102' verbunden.

Mittels der Geräteträgerplattform 10' kann das Instrument 28' in einem sterilen Bereich 130' in einer gewünschten Stellung und Position gehalten werden. Die Kurbelgetriebe 20' und 22' befinden sich in diesem Fall in einem nicht sterilen Bereich, welcher sich auch außerhalb eines Operationsraums befinden könnte.

## Patentansprüche

1. Chirurgische Haltevorrichtung (10) zum Halten eines chirurgischen Instruments (28), mit einem Rahmen (12), mit einer mindestens zwei Lenker (62, 72, 80, 70, 56) umfassenden ersten Lenkeranordnung (20), welche den Rahmen (12) mit einem dem Instrument (28) zugeordneten ersten Gelenkpunkt (24) gelenkig verbindet, und mit einer mindestens zwei Lenker (82, 90, 98, 106, 92) umfassenden zweiten Lenkeranordnung (22), welche den Rahmen (12) mit einem dem Instrument (28) zugeordneten zweiten Gelenkpunkt (26) gelenkig verbindet, wobei die Lenker (62, 72, 80, 70, 56) der ersten Lenkeranordnung (20) relativ zueinander und relativ zum Rahmen (12) um Schwenkachsen (60, 74, 110, 78, 68) schwenkbar gelagert sind und die Schwenkachsen (60, 74, 110, 78, 68) parallel zueinander verlaufen und wobei die beiden Lenkeranordnungen (20, 22) so ausgebildet sind, daß der erste Gelenkpunkt (24) in einer ersten Bewegungsebene (114) und der zweite Gelenkpunkt (26) in einer zweiten Bewegungsebene (116) bewegbar ist, **dadurch gekennzeichnet, daß** die erste Bewegungsebene (114) relativ zur zweiten Bewegungsebene (116) bewegbar ist.

2. Vorrichtung nach Anspruch 1, **dadurch gekennzeichnet, daß** die Lenker (82, 90, 98, 106, 92) der zweiten Lenkeranordnung (22) relativ zueinander und relativ zum Rahmen (12) um Schwenkachsen (88, 100, 108, 104, 94) schwenkbar gelagert sind und daß die Schwenkachsen (88, 100, 108, 104, 94) parallel zueinander verlaufen.

3. Vorrichtung nach Anspruch 1 oder 2, **dadurch gekennzeichnet, daß** die erste Bewegungsebene (114) relativ zur zweiten Bewegungsebene (116) verschwenkbar ist.

4. Vorrichtung nach einem der voranstehenden Ansprüche, **dadurch gekennzeichnet, daß** ein Abstand zwischen der ersten Bewegungsebene (114) und der zweiten Bewegungsebene (116) veränderbar ist.

5. Vorrichtung nach einem der voranstehenden Ansprüche, **dadurch gekennzeichnet, daß** der Rahmen (12) ein erstes und mindestens ein zweites Rahmenelement (14, 16) umfaßt und daß die erste Lenkeranordnung (20) am ersten Rahmenelement (14) und die zweite Lenkeranordnung (22) am zweiten Rahmenelement (16) angeordnet ist.

6. Vorrichtung nach Anspruch 5, **dadurch gekennzeichnet, daß** das erste Rahmenelement (14) relativ zum zweiten Rahmenelement (16) bewegbar gelagert ist.

7. Vorrichtung nach Anspruch 6, **dadurch gekennzeichnet, daß** das erste Rahmenelement (14) relativ zum zweiten Rahmenelement (16) um eine Drehachse (18) verschwenkbar ist.

8. Vorrichtung nach Anspruch 7, **dadurch gekennzeichnet, daß** die Drehachse (18) parallel zu den beiden Bewegungsebenen (114, 116) verläuft.

9. Vorrichtung nach einem der Ansprüche 6 bis 8, **dadurch gekennzeichnet, daß** das erste Rahmenelement (14) relativ zum zweiten Rahmenelement (16) in einer Richtung quer zu der ersten Bewegungsebene (114) verschiebbar ist.

10. Vorrichtung nach einem der voranstehenden Ansprüche, **dadurch gekennzeichnet, daß** die erste Lenkeranordnung (20) fünf relativ zueinander in der ersten Bewegungsebene (114) verschwenkbare Lenker (62, 72, 80, 70, 56) umfaßt.

11. Vorrichtung nach Anspruch 10, **dadurch gekennzeichnet, daß** die fünf Lenker (62, 72, 80, 70, 56) der ersten Lenkeranordnung (20) derart miteinander verbunden sind, daß sie eine geschlossene erste Ringstruktur bilden.

12. Vorrichtung nach einem der Ansprüche 10 oder 11, **dadurch gekennzeichnet, daß** das erste Rahmenelement (14) einen der fünf Lenker (62, 72, 80, 70, 56) der ersten Lenkeranordnung (20) umfaßt.

13. Vorrichtung nach einem der voranstehenden Ansprüche, **dadurch gekennzeichnet, daß** der erste Gelenkpunkt (24) mindestens einem der Lenker (72, 80) der ersten Lenkeranordnung (20) zugeordnet ist.

14. Vorrichtung nach einem der voranstehenden Ansprüche, **dadurch gekennzeichnet, daß** die zweite Lenkeranordnung (22) fünf relativ zueinander in der zweiten Bewegungsebene (116) verschwenkbare Lenker (82, 90, 98, 106, 92) umfaßt.

15. Vorrichtung nach Anspruch 14, **dadurch gekennzeichnet, daß** die fünf Lenker (82, 90, 98, 106, 92) der zweiten Lenkeranordnung (22) derart miteinander verbunden sind, daß sie eine geschlossene zweite Ringstruktur bilden.

16. Vorrichtung nach einem der Ansprüche 14 oder 15, **dadurch gekennzeichnet, daß** das zweite Rahmenelement (16) einen der fünf Lenker (82, 90, 98, 106, 92) der zweiten Lenkeranordnung (22) umfaßt.

17. Vorrichtung nach einem der voranstehenden Ansprüche, **dadurch gekennzeichnet, daß** der zweite Gelenkpunkt (26) mindestens einem der Lenker (98, 106) der zweiten Lenkeranordnung (22) zugeordnet ist.

18. Vorrichtung nach einem der voranstehenden Ansprüche, **dadurch gekennzeichnet, daß** die Schwenkachsen (60, 74, 110, 78, 68) der Lenker (62, 72, 80, 70, 56) der ersten Lenkeranordnung (20) rechtwinklig zur ersten Bewegungsebene (114) verlaufen.

19. Vorrichtung nach einem der voranstehenden Ansprüche, **dadurch gekennzeichnet, daß** die Schwenkachsen (88, 100, 108, 104, 94) der Lenker (82, 90, 98, 106, 92) der zweiten Lenkeranordnung (22) rechtwinklig zur zweiten Bewegungsebene (116) verlaufen.

20. Vorrichtung nach einem der voranstehenden Ansprüche, **dadurch gekennzeichnet, daß** mindestens ein Lenkerantrieb (64, 66, 86, 96) zum Bewegen mindestens eines am Rahmen (12) gelagerten Lenkers (62, 70, 90, 92) einer der beiden Lenkeranordnungen (20, 22) vorgesehen ist.

21. Vorrichtung nach Anspruch 20, **dadurch gekennzeichnet, daß** jeder der beiden Lenkeranordnungen (20, 22) zwei Lenkerantriebe (64, 66; 86, 96) zugeordnet sind.

22. Vorrichtung nach einem der Ansprüche 20 oder 21, **dadurch gekennzeichnet, daß** der mindestens eine Lenkerantrieb ein Rotationsantrieb (64, 66, 86, 96) zum Schwenken des mindestens einen am Rahmen (12) gelagerten Lenkers (62, 70, 90, 92) relativ zum Rahmen (12) ist.

23. Vorrichtung nach einem der Ansprüche 20 bis 22, **dadurch gekennzeichnet, daß** der mindestens eine Lenkerantrieb (64, 66, 86, 96) am Rahmen (12) angeordnet ist.

24. Vorrichtung nach einem der Ansprüche 20 bis 23, **dadurch gekennzeichnet, daß** die Lenkerantriebe (64, 66, 86, 96) unabhängig voneinander ansteuerbar sind.

25. Vorrichtung nach einem der voranstehenden Ansprüche, **dadurch gekennzeichnet, daß** jede der beiden Lenkeranordnungen (20, 22) ein Halteelement (120, 122) trägt und daß jedem Halteelement (120, 122) einer der beiden Gelenkpunkte (24, 26) zugeordnet ist.

26. Vorrichtung nach Anspruch 25, **dadurch gekennzeichnet, daß** das mindestens eine Halteelement (120, 122) lösbar mit einem der Lenker (80, 106) verbindbar ist.

27. Vorrichtung nach einem der Ansprüche 25 oder 26, **dadurch gekennzeichnet, daß** jedes Halteelement (120, 122) eine Sterilschnittstelle (124, 126) umfaßt.

28. Vorrichtung nach einem der voranstehenden Ansprüche, **dadurch gekennzeichnet, daß** mindestens eine der beiden Lenkeranordnungen (20, 22) zwei sich überkreuzende Lenker (62, 70; 90, 92) umfaßt.

29. Vorrichtung nach einem der voranstehenden Ansprüche, **dadurch gekennzeichnet, daß** die Vorrichtung (10) nur Drehgelenke zum Bewegen der Lenker (62, 72, 80, 70, 56; 82, 90, 98, 106, 92) und der Gelenkpunkte (24, 26) relativ zueinander umfaßt.

30. Vorrichtung nach einem der voranstehenden Ansprüche, **dadurch gekennzeichnet, daß** der erste und/oder der zweite Gelenkpunkt ein Mehrfreiheitsgradgelenk (24, 26) umfaßt.

31. Vorrichtung nach Anspruch 30, **dadurch gekennzeichnet, daß** das Mehrfreiheitsgradgelenk ein Kugelgelenk (24, 26) ist.

32. Vorrichtung nach Anspruch 30, **dadurch gekennzeichnet, daß** das Mehrfreiheitsgradgelenk (24, 26) in Form eines Kreuzgelenks ausgebildet ist.

33. Vorrichtung nach einem der voranstehenden Ansprüche, **dadurch gekennzeichnet, daß** das Instrument (28) an mindestens einem der beiden Gelenkpunkte (24, 26) verschiebbar gehalten ist.

34. Vorrichtung nach Anspruch 33, **dadurch gekennzeichnet, daß** eine Führung für das Instrument (28) vorgesehen ist und daß die beiden Gelenkpunkte (24, 26) an der Führung angeordnet sind.

## Claims

1. Surgical holding device (10) for holding a surgical instrument (28), with a frame (12), with a first linkage (20) comprising at least two links (62, 72, 80, 70, 56), which articulatedly connects the frame (12) to a first articulation point (24) associated with the instrument (28), and with a second linkage (22) comprising at least two links (82, 90, 98, 106, 92), which articulatedly connects the frame (12) to a second articulation point (26) associated with the instrument (28), wherein the links (62, 72, 80, 70, 56) of the first linkage (20) are mounted for pivotal movement about pivot axes (60, 74, 110, 78, 68) relative to one another and relative to the frame (12), and the pivot axes (60, 74, 110, 78, 68) extend parallel to one another, and wherein the two linkages (20, 22) are configured such that the first articulation point (24) is movable in a first plane of movement (114) and the second articulation point (26) in a second plane of movement (116), **characterized in that** the first plane of movement (114) is movable relative to the second plane of movement (116).

2. Device in accordance with claim 1, **characterized in that** the links (82, 90, 98, 106, 92) of the second linkage (22) are mounted for pivotal movement about pivot axes (88, 100, 108, 104, 94) relative to one another and relative to the frame (12), and the pivot axes (88, 100, 108, 104, 94) extend parallel to one another.

3. Device in accordance with claim 1 or 2, **characterized in that** the first plane of movement (114) is pivotable relative to the second plane of movement (116).

4. Device in accordance with any one of the preceding claims, **characterized in that** a spacing between the first plane of movement (114) and the second plane of movement (116) is alterable.

5. Device in accordance with any one of the preceding claims, **characterized in that** the frame (12) comprises a first and at least a second frame element (14, 16), and **in that** the first linkage (20) is arranged on the first frame element (14) and the second linkage (22) on the second frame element (16).

6. Device in accordance with claim 5, **characterized in that** the first frame element (14) is mounted so as to be movable relative to the second frame element (16).

7. Device in accordance with claim 6, **characterized in that** the first frame element (14) is pivotable about an axis of rotation (18) relative to the second frame element (16).

8. Device in accordance with claim 7, **characterized in that** the axis of rotation (18) extends parallel to the two planes of movement (114, 116).

9. Device in accordance with any one of claims 6 to 8, **characterized in that** the first frame element (14) is displaceable in a direction transverse to the first plane of movement (114) relative to the second frame element (16).

10. Device in accordance with any one of the preceding claims, **characterized in that** the first linkage (20) comprises five links (62, 72, 80, 70, 56) pivotable relative to one another in the first plane of movement (114).

11. Device in accordance with claim 10, **characterized in that** the five links (62, 72, 80, 70, 56) of the first linkage (20) are connected to one another so as to form a closed first ring structure.

12. Device in accordance with claim 10 or 11, **characterized in that** the first frame element (14) comprises one of the five links (62, 72, 80, 70, 56) of the first linkage (20).

13. Device in accordance with any one of the preceding claims, **characterized in that** the first articulation point (24) is associated with at least one of the links (72, 80) of the first linkage (20).

14. Device in accordance with any one of the preceding claims, **characterized in that** the second linkage (22) comprises five links (82, 90, 98, 106, 92) pivotable relative to one another in the second plane of movement (116).

15. Device in accordance with claim 14, **characterized in that** the five links (82, 90, 98, 106, 92) of the second linkage (22) are connected to one another so as to form a closed second ring structure.

16. Device in accordance with claim 14 or 15, **characterized in that** the second frame element (16) comprises one of the five links (82, 90, 98, 106, 92) of the second linkage (22).

17. Device in accordance with any one of the preceding claims, **characterized in that** the second articulation point (26) is associated with at least one of the links (98, 106) of the second linkage (22).

18. Device in accordance with any one of the preceding claims, **characterized in that** the pivot axes (60, 74, 110, 78, 68) of the links (62, 72, 80, 70, 56) of the first linkage (20) extend at right angles to the first plane of movement (114).

19. Device in accordance with any one of the preceding claims, **characterized in that** the pivot axes (88, 100, 108, 104, 94) of the links (82, 90, 98, 106, 92) of the second linkage (22) extend at right angles to the second plane of movement (116).

20. Device in accordance with any one of the preceding claims, **characterized in that** at least one link drive (64, 66, 86, 96) is provided for moving at least one link (62, 70, 90, 92), mounted on the frame (12), of one of the two linkages (20, 22).

21. Device in accordance with claim 20, **characterized in that** each of the two linkages (20, 22) has two link drives (64, 66, 86, 96) associated with it.

22. Device in accordance with claim 20 or 21, **characterized in that** the at least one link drive is a rotational drive (64, 66, 86, 96) for pivoting the at least one link (62, 70, 90, 92) mounted on the frame (12) relative to the frame (12).

23. Device in accordance with any one of claims 20,to 22, **characterized in that** the at least one link drive (64, 66, 86, 96) is arranged on the frame (12).

24. Device in accordance with any one of claims 20,to 23, **characterized in that** the link drives (64, 66, 86, 96) are activatable independently of one another.

25. Device in accordance with any one of the preceding claims, **characterized in that** each of the two linkages (20, 22) carries a holding element (120, 122), and each holding element (120, 122) has one of the two articulation points (24, 26) associated with it.

26. Device in accordance with claim 25, **characterized in that** the at least one holding element (120, 122) is releasably connectable to one of the links (80, 106).

27. Device in accordance with claim 25 or 26, **characterized in that** each holding element (120, 122) comprises a sterile interface (124, 126).

28. Device in accordance with any one of the preceding claims, **characterized in that** at least one of the two linkages (20, 22) comprises two intersecting links (62, 70; 90, 92).

29. Device in accordance with any one of the preceding claims, **characterized in that** the device (10) comprises only rotary joints for moving the links (62, 72, 80, 70, 56; 82, 90, 98, 106, 92) and the articulation points (24, 26) relative to one another.

30. Device in accordance with any one of the preceding claims, **characterized in that** the first and/or the second articulation point comprises a joint with multiple degrees of freedom (24, 26).

31. Device in accordance with claim 30, **characterized in that** the joint with multiple degrees of freedom is a ball-and-socket joint (24, 26).

32. Device in accordance with claim 30, **characterized in that** the joint with multiple degrees of freedom (24, 26) is in the form of a universal joint.

33. Device in accordance with any one of the preceding claims, **characterized in that** the instrument (28) is held displaceably at at least one of the two articulation points (24, 26).

34. Device in accordance with claim 33, **characterized in that** a guide is provided for the instrument (28), and the two articulation points (24, 26) are arranged on the guide.

## Revendications

1. Dispositif chirurgical de maintien (10), destiné au maintien d'un instrument chirurgical (28), avec un cadre (12), avec un premier dispositif de guidage (20) comportant au moins deux bras (62, 72, 80, 70, 56), lequel relie par articulation le cadre (12) à un premier point d'articulation (24) associé à l'instrument (28), et avec un deuxième dispositif de guidage (22) comportant au moins deux bras (82, 90, 98, 106, 92), lequel relie par articulation le cadre (12) à un deuxième point d'articulation (26) associé à l'instrument (28), les bras (62, 72, 80, 70, 56) du premier dispositif de guidage (20) étant montés de manière à pivoter autour d'axes de pivotement (60, 74, 110, 78, 68) l'un par rapport à l'autre et par rapport au cadre (12), et les axes de pivotement (60, 74, 110, 78, 68) s'étendant parallèlement l'un à l'autre, et les deux dispositifs de guidage (20, 22) étant réalisés de telle manière que le premier point d'articulation (24) est mobile sur un premier plan de déplacement (114) et le deuxième point d'articulation (26) est mobile sur un deuxième plan de déplacement (116), **caractérisé en ce que** le premier plan de déplacement (114) est mobile par rapport au deuxième plan de déplacement (116).

2. Dispositif selon la revendication 1, **caractérisé en ce que** les bras (82, 90, 98, 106, 92) du deuxième dispositif de guidage (22) sont montés de manière à pivoter autour d'axes de pivotement (88, 100, 108, 104, 94) l'un par rapport à l'autre et par rapport au cadre (12), et **en ce que** les axes de pivotement (88, 100, 108, 104, 94) s'étendent parallèlement l'un à l'autre.

3. Dispositif selon la revendication 1 ou 2, **caractérisé en ce que** le premier plan de déplacement (114) est mobile par rapport au deuxième plan de déplacement (116).

4. Dispositif selon l'une des revendications précédentes, **caractérisé en ce qu'**un espacement peut être modifié entre le premier plan de déplacement (114) et le deuxième plan de déplacement (116).

5. Dispositif selon l'une des revendications précédentes, **caractérisé en ce que** le cadre (12) comprend un premier et au moins un deuxième élément de cadre (14, 16), et **en ce que** le premier dispositif de guidage (20) est disposé sur le premier élément de cadre (14) et le deuxième dispositif de guidage (22) sur le deuxième élément de cadre (16).

6. Dispositif selon la revendication 5, **caractérisé en ce que** le premier élément de cadre (14) est monté de manière à être mobile par rapport au deuxième élément de cadre (16).

7. Dispositif selon la revendication 6, **caractérisé en ce que** le premier élément de cadre (14) est pivotant autour d'un axe de rotation (18) par rapport au deuxième élément de cadre (16).

8. Dispositif selon la revendication 7, **caractérisé en ce que** l'axe de rotation (18) s'étend parallèlement aux deux plans de déplacement (114, 116).

9. Dispositif selon l'une des revendications 6 à 8, **caractérisé en ce que** le premier élément de cadre (14) est mobile dans une direction perpendiculaire au premier plan de déplacement (114) par rapport au deuxième élément de cadre (16).

10. Dispositif selon l'une des revendications précédentes, **caractérisé en ce que** le premier dispositif de guidage (20) comporte cinq bras (62, 72, 80, 70, 56) pivotant l'un par rapport à l'autre sur le premier plan de déplacement (114).

11. Dispositif selon la revendication 10, **caractérisé en ce que** les cinq bras (62, 72, 80, 70, 56) du premier dispositif de guidage (20) sont reliés l'un à l'autre de manière à former une première structure annulaire fermée.

12. Dispositif selon l'une des revendications 10 ou 11, **caractérisé en ce que** le premier élément de cadre (14) comporte un des cinq bras (62, 72, 80, 70, 56) du premier dispositif de guidage (20).

13. Dispositif selon l'une des revendications précédentes, **caractérisé en ce que** le premier point d'articulation (24) est associé à au moins un des bras (72, 80) du premier dispositif de guidage (20).

14. Dispositif selon l'une des revendications précédentes, **caractérisé en ce que** le deuxième dispositif de guidage (22) comporte cinq bras (82, 90, 98, 106, 92) pivotant l'un par rapport à l'autre sur le deuxième plan de déplacement (116).

15. Dispositif selon la revendication 14, **caractérisé en ce que** les cinq bras (82, 90, 98, 106, 92) du deuxième dispositif de guidage (22) sont reliés l'un à l'autre de manière à former une deuxième structure annulaire fermée.

16. Dispositif selon l'une des revendications 14 ou 15, **caractérisé en ce que** le deuxième élément de cadre (16) comprend un des cinq bras (82, 90, 98, 106, 92) du deuxième dispositif de guidage (22).

17. Dispositif selon l'une des revendications précédentes, **caractérisé en ce que** le deuxième point d'articulation (26) est associé à au moins un des bras (98, 106) du deuxième dispositif de guidage (22).

18. Dispositif selon l'une des revendications précédentes, **caractérisé en ce que** les axes de pivotement (60, 74, 110, 78, 68) des bras (62, 72, 80, 70, 56) du premier dispositif de guidage (20) s'étendent perpendiculairement au premier plan de déplacement (114).

19. Dispositif selon l'une des revendications précédentes, **caractérisé en ce que** les axes de pivotement (88, 100, 108, 104, 94) des bras (82, 90, 98, 106, 92) du deuxième dispositif de guidage (22) s'étendent perpendiculairement au deuxième plan de déplacement (116).

20. Dispositif selon l'une des revendications précédentes, **caractérisé en ce qu'**il est prévu au moins un entraînement de bras (64, 66, 86, 96) pour le déplacement d'au moins un bras (62, 70, 90, 92), monté sur le cadre (12), d'un des deux dispositifs de guidage (20, 22).

21. Dispositif selon la revendication 20, **caractérisé en ce qu'**à chacun des deux dispositifs de guidage (20, 22) sont associés deux entraînements de bras (64, 66 ; 86, 96).

22. Dispositif selon l'une des revendications 20 ou 21, **caractérisé en ce que** le ou les entraînements de bras est un actionneur rotatif (64, 66, 86, 96) pour le pivotement par rapport au cadre (12) du ou des bras (62, 70, 90, 92) montés sur le cadre (12).

23. Dispositif selon l'une des revendications 20 à 22, **caractérisé en ce que** le ou les entraînements de bras (64, 66, 86, 96) est ou sont disposés sur le cadre (12).

24. Dispositif selon l'une des revendications 20 à 23, **caractérisé en ce que** les entraînements de bras (64, 66, 86, 96) peuvent être commandés indépendamment l'un de l'autre.

25. Dispositif selon l'une des revendications précédentes, **caractérisé en ce que** chacun des deux dispositifs de guidage (20, 22) supporte un élément de maintien (120, 122), et **en ce qu'**à chaque élément de maintien (120, 122) est associé un des deux points d'articulation (24, 26).

26. Dispositif selon la revendication 25, **caractérisé en ce que** le ou les éléments de maintien (120, 122) peut ou peuvent être raccordés de manière amovible à un des bras (80, 106).

27. Dispositif selon l'une des revendications 25 ou 26, **caractérisé en ce que** chaque élément de maintien (120, 122) comprend une interface stérile (124, 126).

28. Dispositif selon l'une des revendications précédentes, **caractérisé en ce qu'**au moins un des deux dispositifs de guidage (20, 22) comporte deux bras (62, 70 ; 90, 92) qui se croisent.

29. Dispositif selon l'une des revendications précédentes, **caractérisé en ce que** le dispositif (10) ne comprend que des articulations à pivot pour le déplacement des bras (62, 72, 80, 70, 56 ; 82, 90, 98, 106, 92) et des points d'articulation (24, 26) l'un par rapport à l'autre.

30. Dispositif selon l'une des revendications précédentes, **caractérisé en ce que** le premier et/ou le deuxième point d'articulation comportent une articulation à plusieurs degrés de liberté (24, 26).

31. Dispositif selon la revendication 30, **caractérisé en ce que** l'articulation à plusieurs degrés de liberté est une articulation à rotule (24, 26).

32. Dispositif selon la revendication 30, **caractérisé en ce que** l'articulation à plusieurs degrés de liberté (24, 26) est réalisée sous la forme d'une articulation à cardan.

33. Dispositif selon l'une des revendications précédentes, **caractérisé en ce que** l'instrument (28) est maintenu de manière à être mobile sur au moins un des deux points d'articulation (24, 26).

34. Dispositif selon la revendication 33, **caractérisé en ce qu'**il est prévu un guidage pour l'instrument (28), et **en ce que** les deux points d'articulation (24, 26) sont disposés sur le guidage.
